# EUROPEAN PATENT SPECIFICATION

(11) **EP 2 693 958 B1**
(45) Date of publication and mention of the grant of the patent: **06.04.2016**
(21) Application number: 12748528.2
(22) Date of filing: 10.04.2012
(51) Int. Cl.: A61B 17/17

(54) **DRILLING MASK FOR IMPLANTING A TRANSPEDICULAR SCREW**
BOHRLEHRE ZUR IMPLANTATION EINER TRANSPEDIKELSCHRAUBE
MASQUE DE PERÇAGE POUR IMPLANTER UNE VIS TRANSPÉDICULAIRE

(30) Priority: 08.04.2011 IT PI20110040
(43) Date of publication of application: 12.02.2014
(73) Proprietor: UNIVERSITA' DI PISA, 56126 Pisa (IT)
(72) Inventor: CONDINO, Sara, I-56124 Pisa (IT); FERRARI, Vincenzo, I-56124 Pisa (IT); PARCHI, Paolo, I-56124 Pisa (IT); LISANTI, Michele, I-56124 Pisa (IT)
(74) Representative: Celestino, Marco
(86) International application number: PCT/IB2012/051747
(87) International publication number: WO 2012/140569

(56) References cited:
- CN-A- 101 536 929
- DE-U1- 20 301 260
- FR-A1- 2 713 473
- US-A- 4 907 577
- US-A1- 2009 105 761
- LU SHENG ET AL: "A novel patient-specific navigational template for cervical pedicle screw placement.", SPINE 15 DEC 2009, vol. 34, no. 26, 15 December 2009 (2009-12-15), pages E959-E966, ISSN: 1528-1159
- LU SHENG ET AL: "A novel computer-assisted drill guide template for lumbar pedicle screw placement: a cadaveric and clinical study.", THE INTERNATIONAL JOURNAL OF MEDICAL ROBOTICS + COMPUTER ASSISTED SURGERY : MRCAS JUN 2009, vol. 5, no. 2, June 2009 (2009-06), pages 184-191, ISSN: 1478-596X

## Description

### Field of the invention

The present invention relates to the medical field, with application in orthopaedic surgery and, in particular, in the treatment of diseases of the spinal column, and more precisely it relates to a drilling mask for drilling holes for surgical operations in the orthopaedic field.

### Description of the prior art

As well known, transpedicular screws in the vertebral arthrodesis surgery are used to treat many affections of the spinal column caused for example by neoplasms, alteration of development, congenital, traumatic and degenerative diseases.

The steps necessary for arranging transpedicular screws represents a complex technique owing to the variability in the shape, size and orientation of the vertebral pedicles and owing to their vicinity with the neurological structures. For example, at a thoracic level the introduction of the screws is particularly complicated owing to the reduced size of the pedicles and to their vicinity with the pleura and the spinal bone marrow.

If positioned in an ideal way a transpedicular screw should be completely contained in the vertebral pedicle avoiding to protrude in the vertebral space and in the conjugation foramen. On the contrary, a wrong location can determine an instability of the treated vertebral segment and neurological injuries, such as paralysis of several nervous roots, paraplegia, sphyncteric troubles, incontinence, with serious damages to the quality of life of the patients and high social costs. In particular, when a screw violates the pedicle cortical layer there is the risk of injuries to the spinal bone marrow and to the nervous roots.

The spine stabilization with transpedicular screws presents many advantages with respect to the stabilization with vertebral hooks, since it has a higher biomechanical stability that permits stabilizing and at the same time correcting the vertebral deformity, especially when there is a need to include in the arthrodesis area shorter spine segments. Furthermore, it avoids introducing instruments in the vertebral channel, thus decreasing the risk of neurological damage and is independent from the soundness of the vertebral discs and then can be done also in conditions as cancer or traumatic diseases where the use of the hooks is not recommended.

The presently known techniques for guiding the transpedicular screws are the freehand positioning, the fluoroscopy and the use of surgical navigators.

In the "freehand" positioning technique the rate of positioning errors is about 20%, and is however quite underestimated since often positioning errors are completely asymptomatic and can be detected only by a TC study.

Intraoperatory fluoroscopy is a commonly used technique for guiding the surgeon when implanting transpedicular screws. However, the use of a fluoroscopic control determines only a low improvement in the precision in positioning the screws, since it is capable of providing only projective images, and when implanting the screw the surgeon must continuously alternate controls in different projections (frontal-rear and lateral-lateral), with subsequent waste of time and exposition of the surgical team and the patient to ionizing radiations.

The use of other computer-aided imaging systems for locating the transpedicular screws is uncommon, since it has limits due to the high cost of the instrumentations, by the substantive increase of the operation time and by the need in most cases of positioning in the patient optical markers (for adjusting the pre-operatory model on the anatomy of the patient) that would make much more complex the surgical operation. The use of TC during the surgical operation would make it possible to obtain guiding images once positioned the patient and would eliminate the problem of the registration, and would reduce significantly the errors with respect to the previous methods, but would expose the patient to high doses of ionizing radiations and would also present extraction difficulties, with high costs, and for this reason is quite uncommon in these operations.

Furthermore, devices are known that provide the use of tubular screws whose introduction is guided by means of Kirschner wires. In this case, also the Kirschner wires can be guided with the above described techniques.

Drilling masks and/or cutting masks are also known for implanting bone screws or synthetic grafts, in particular when making orthodontic surgery implants, as described in WO03071972A1, and in the maxillofacial surgery. These masks, in case of large traumas, are a very useful solution for achieving the maximum functional and aesthetical results for the patient. Recently, similar solutions have been developed for the knee surgery, as described in WO2010033431A1.

The application of the drilling masks of the prior art for the application of transpedicular screws, would not be effective. In fact technical problems would arise capable of affecting the quality of the operation, concerning: increased invasivity, for the need detaching the soft tissues from all the spine section; low precision when implanting the screws, owing to the low precision of the support of the drilling masks; impossibility to guide the screws by a Kirschner wire that can be used as guide for tubular screws.

The latter problem of the known drilling masks, which is due to the problem of removing the drilling mask after drilling the hole in order to drive the screws in the pedicle, is particularly relevant. In fact, since it is impossible to guide the screws by a Kirschner wire keeping the mask in position, the surgeon looses completely the position of the hole made that has to be found again shifting away the soft tissues that are hiding it. On the other hand, if the drilling mask is not withdrawn before the introduction of the screw (that could be advantageously a tubular screw guided by the Kirschner wire) the mask extraction would be impossible after driving the tubular screw.

In US4907577A a drilling mask is described comprising a L-shaped support body with a guide means and a adjustable positioning means for adjusting the drilling position. The preamble of claim 1 is based on this disclosure.

Other solutions of prior art are also disclosed in DE20301260, US2009/105761 and FR2713473.

The above described drilling mask, however, does not allow a precise definition of the drilling axis. In fact, the arrangement of the guide channels for assisting the drilling operation is effected by the surgeon in an empirical way and during the operation. Such involves the risk of positioning errors and then of drilling errors in the bone.

### Summary of the invention

It is therefore a feature of the present invention to provide a drilling mask for drilling the bone and guiding transpedicular screws with extreme precision in the pedicles.

It is another feature of the present invention to provide a drilling mask that can be removed only at the end of the step of introducing the transpedicular screws, in particular tubular and non tubular screws.

It is also a feature of the present invention to provide a drilling mask that is suitable for perfectly fitting the spinal column and the corresponding bone profile, in order to increase the drilling stability.

It is another feature of the present invention to provide a drilling mask that is cheap and at the same time improves the precision of putting the transpedicular screws and does not cause excessive X-ray applications for the patient by the surgeon.

It is still a feature of the present invention to provide a drilling mask for being used without increased invasivity, i.e. without a need of detaching all the tissues from the spine in the operation area.

It is still particular feature of the present invention to provide a drilling mask to minimize the amount of tissues to remove/detach and at the same time provides a steady and deterministic support.

These and other objects are achieved by a drilling .mask that is adapted for application to a spine section for implanting a transpedicular screw, said spine section having a vertebral body with a spinous process, a vertebral lamina and an articular process with a relative pedicle, said drilling mask comprising:
- a guide body having a distal surface and a proximal surface, opposite to said distal surface and facing towards said spine section,
- a reference element obtained on said proximal surface and arranged to contact said spinous process,
- a guide channel having an inlet at said distal surface and arranged to work as drilling guide for drilling said pedicle and implanting said transpedicular screw;
- a plurality of support elements made on said proximal surface, associated with said reference element and arranged to contact side parts of said spine section,
wherein said guide body comprises at least one reference portion, comprising said reference element, configured to be put at said spinous process, and a lateral guide portion, cooperating with said reference portion, and comprising said guide channel for drilling said pedicle, wherein said reference portion and said lateral guide portion are mutually coupled to each other through releasable mutual engagement means, in order to move from a use configuration, where said reference portion and said lateral guide portion are connected to each other for introducing said transpedicular screw, to a extraction configuration, where said reference portion and said lateral guide portion are disassembled from each other for removal of said lateral guide portion in a direction substantially parallel with respect to the axis of said transpedicular screw and for removal of said reference portion in a direction substantially parallel to said spinous process,
and wherein said reference element is an element having a groove arranged to engage and bridge said spinous process.

This way, through a guide body comprised of at least two portions mutually coupled to each other, it is possible, after driving the transpedicular screw, to remove the portions of the drilling mask in a direction substantially orthogonal towards the above with respect to their arrangement onto the spine section. In fact, the axis of the spinous process and of the transpedicular screw cannot coincide, and owing to the separation between the lateral guide portion and the reference portion, it is possible to extract them separately, each following its axis.

In addition, the reference element that engages with the spinous process is extremely useful for guiding the drilling mask with a correct orientation. Integrally to the other support elements, in addition to the stability of the drilling mask, also a way checking its correct location is achieved.

More in particular, the portion which comprises the guide channel is arranged at an angle with respect to the portion which comprises the reference element arranged in contact with the spinous process, such that once inserted the screw it is possible to extract the mask portion in a way substantially parallel with respect to the axis of the screw.

This solution is particularly useful also if tubular screws are used guided by a Kirschner wire. In this case, in fact, it is possible to firstly put in the pedicle a Kirschner wire that can be used as guide for tubular screws or directly for guiding the final screws (tubular or solid). Alternatively, this solution allows in addition to the removal also the change of the lateral guide portions for making holes and then for guiding the screws.

In particular, said guide body comprises two lateral guide portions arranged at opposite sides with respect to said reference portion and equipped with a respective guide channel, each of the lateral guide portions being releasably connected to the reference portion, in such a way that it can pass from said use configuration to said extraction configuration. This way, the drilling mask is modular and the lateral portion can be replaced responsive to the kind of transpedicular screw that has to be implanted.

In particular, said plurality of support elements is arranged to contact side parts of said spine section and comprise at least one couple of support elements for said vertebral lamina arranged symmetrically with respect to said spinous process, at least one couple of support elements on said articular process, which are arranged symmetrically with respect to said spinous process associated with said guide channels configured to be put at a respective pedicle. This way, the drilling mask presents a plurality of support elements in order to reduce to the minimum the tissues to remove/detach and at the same time provides a steady and deterministic support, without false support points, i.e. all the support elements are in a steady position of the spine section and do not rest on the tissues.

Advantageously, said reference element and said couple of support elements on said vertebral lamina and on said articular processes and said guide channel are connected to each other by connection elements, in order to form a support bar arranged to keep a precise relative position between said supports, said reference element and said guide channel. This way, the two couples of support elements provide a stability to the structure and allow the correct arrangement of the same, since they allow a redundant verification of the position of the support elements for determining false positioning due to the presence of residues of soft tissue. In other words, if for example the mask is stable, but the surgeon verifies that a support has moved upwards, it means that the positioning is not correct and that it is necessary to check whether there are soft tissue residues under the other support elements.

In particular, said guide channel can comprise a reduction element, in particular a bush, configured to be put in said guide channel, in order to reduce the diameter for use of transpedicular screws that enter precisely in said reduction element. This way, it is possible to adapt the guide channel responsive to the diameter of the transpedicular screws used, in order to obtain a higher introduction precision.

Advantageously, said drilling mask is obtained starting from imaging means that obtain a volumetric image of said spine section, and a CAD modelling means, for modelling the drilling mask on the basis of said volumetric image, such that said reference element on said spinous process, said support element on said vertebral lamina, and said support element on said articular process, fit the bone profile, in order to obtain a support stability for implanting said transpedicular screw. This way, the drilling mask can be positioned on the corresponding vertebra of the patient after a surgical access of size similar to a traditional operation under fluoroscopy or freehand operation.

The couple of vertebral support elements on the vertebral lamina and on the articular process of the mask are located after having shifted the muscular tissue located underneath, whereas for the reference spinous element no additional steps are necessary, with respect to the traditional operation. Once effected the correct arrangement of the drilling mask and then of the guide channel, the surgeon can proceed, according to the screws used, for drilling the pedicle and then introducing a Kirschner wire for causing a tubular screw to slide on it, or proceeding directly to the guide of the screw, putting the channel guide for implanting screws.

Preferably, said imaging means is adapted to obtain radiographic volumetric images, in particular tomography computerized, magnetic resonance or angiographic three-dimensional rotational images.

In particular, said CAD modelling means provides a means for segmenting said radiographic volumetric images so that said reference element and said support elements have a profile that is the negative of the bone profile obtained by said segmentation, thus contributing to increase the stability and the correct positioning.

### Brief description of the drawings

The invention will be now shown with the following description of an exemplary embodiment thereof, exemplifying but not limitative, with reference to the attached drawings in which:
- Fig. 1 shows a perspective view of a drilling mask, according to the invention, with a guide body mounted to a spine section for implanting a transpedicular screw;
- Fig. 2 shows in a perspective enlarged view a reference portion and a lateral guide portion of the guide body and connected to each other through mutual engagement means that allow the removal of the drilling mask;
- Fig. 3 shows a perspective view of the drilling mask comprising the reference portion to which two lateral guide portions are coupled, which comprise support elements on respective side parts of the spine section;
- Fig. 4 shows a perspective view of the drilling mask Fig. 3 that depicts the possibility of separating the lateral guide portions from the reference portion for removal of the drilling mask;
- Fig. 5 shows a perspective view of the drilling mask of Fig. 3 in a use configuration for introducing the transpedicular screws, this version provides, furthermore, connection elements between the many support elements;
- Fig. 6 shows a perspective view of the drilling mask in a configuration for removing one of the lateral guide portions from the spine section after the introduction of the transpedicular screw;
- Fig. 7 shows a perspective view of the drilling mask comprising the connection elements between the support elements;
- Fig. 8, 8A and 8B show a perspective view of different lateral guide portions and of the reference portion that depicts the mutual engagement means between the parts;
- Fig. 9 shows a perspective view a reduction element to bush for adapting the kind of screw to put in the guide channel of the guide portions;
- finally, Fig. 10 shows a perspective view of the solution without reduction element which allows the use of a tubular screw guided by a Kirschner wire.

### Description of a preferred exemplary embodiment

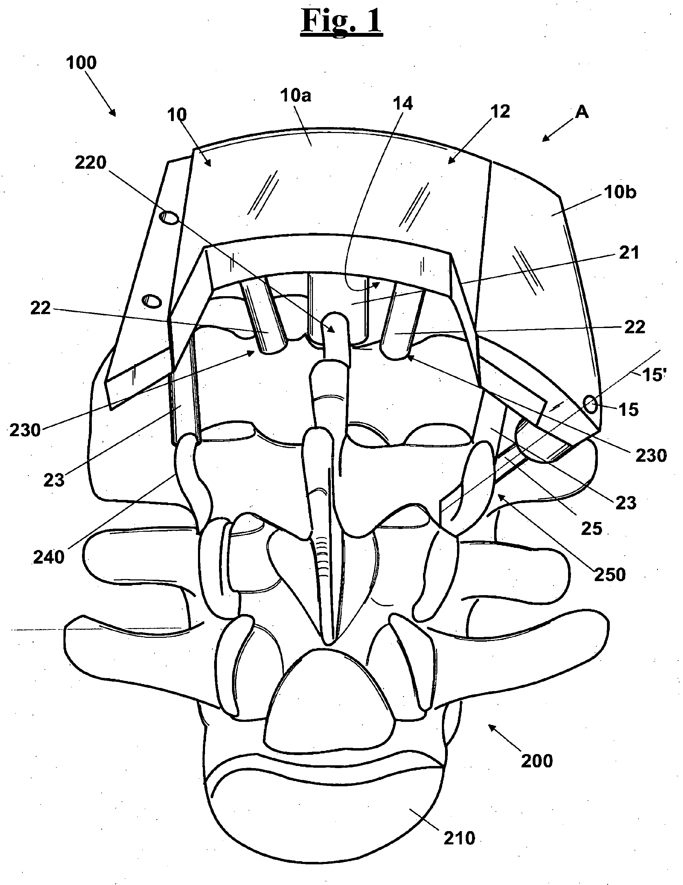

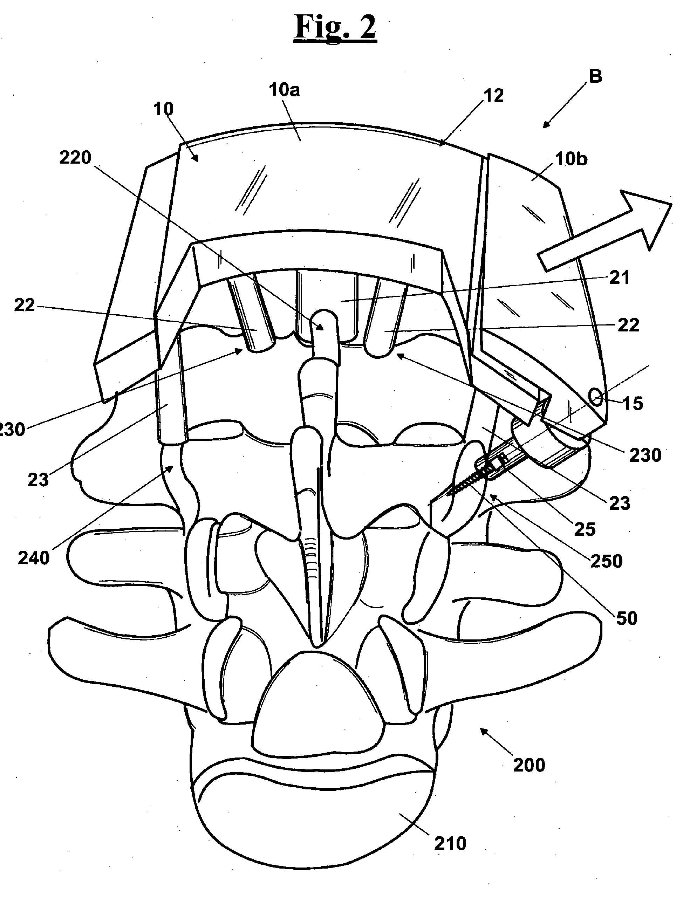

With reference to Fig. 1, a drilling mask 100 is provided for application to a spine section 200 for implanting a transpedicular screw 50 (Fig.2). In particular, spine section 200 comprises a vertebral body 210, a spinous process 220, a vertebral lamina 230 and an articular process 240 with a relative pedicle 250, which define a predetermined bone profile 260.

Drilling mask 100, according to the invention, comprises a guide body 10 with a distal or upper surface 12, distally arranged with respect to spine section 200, and a proximal or lower 14 surface, opposite to the distal surface 12 and facing towards spine section 20b.

In particular, guide body 10 comprises a reference element 21 obtained on the proximal surface 14 and arranged to contact spinous process 220, and a guide channel 25 that extends starting from the distal surface 12 is arranged to work as drilling guide for drilling the pedicle 250 and implanting transpedicular screw 50. In addition, guide body 10 comprises a plurality of support elements 22, 23 made on the proximal surface 14 and associated with a reference element 21, arranged to contact side parts of the spine section 220.

More in particular, guide body 10 comprises at least one reference portion 10a, which represents the central portion of the mask, equipped with reference element 21, and configured to be put at spinous process 220. Furthermore, guide body 10 comprises at least one lateral guide portion 10b, cooperating with reference portion 10a, which comprises guide channel 25 for drilling the pedicle and the support elements 22, 23 laterally with respect to spinous process 220.

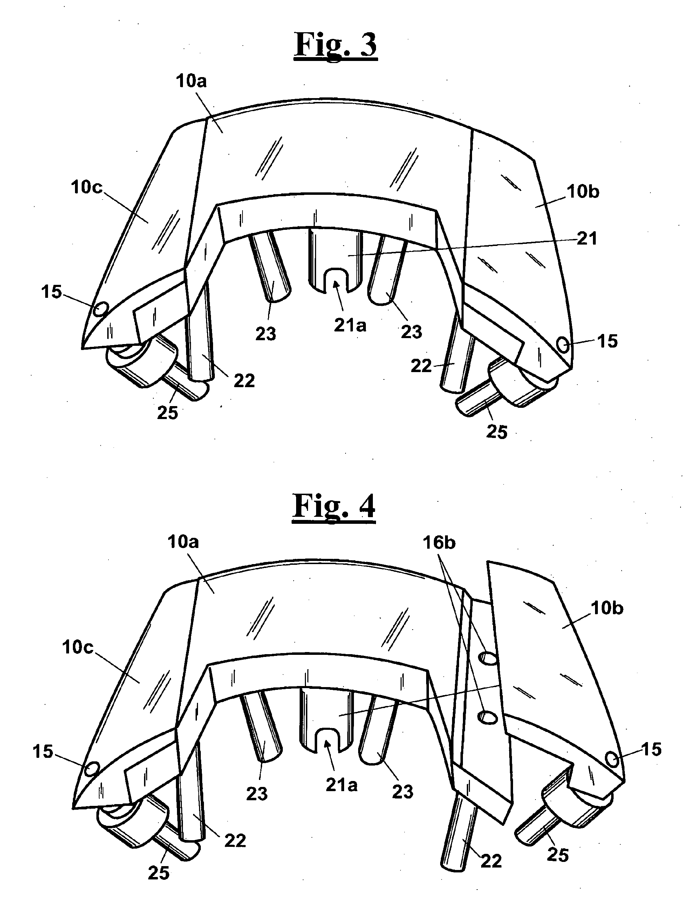

Reference portion 10a and lateral guide portion 10b are mutually coupled to each other through releasable mutual engagement means 16, in order to move them from a use configuration to (Fig.1), where reference portion 10a and lateral guide portion 10b (portion 10c is not present and can be provided as shown in Fig.3-Fig.8) are connected to each other for introducing transpedicular screw 50, to an extraction configuration B (Fig.2). In this extraction configuration B, reference portion 10a and lateral guide portion 10b are disassembled from each other, for removal of lateral guide portion 10b in a direction substantially parallel with respect to the axis 15' of transpedicular screw 50, and for removal of reference portion 10a in a direction substantially parallel to spinous process 220. In particular, the extraction of reference portion 10a is carried out according to a direction which is orthogonal to the front plane of the spinous process.

This way, through a guide body 10 that comprises at least two portions mutually coupled to each other 10a and 10b (or also 10c), it is possible, after having driven transpedicular screw 50, to remove the portions of the drilling mask in a direction substantially orthogonal with respect to their arrangement on spine section 200. More in particular, lateral guide portion 10b or 10c that comprises the guide channel 25 is arranged at an angle with respect to reference portion 10a that comprises reference element 21 arranged in contact with spinous process 220, such that once inserted transpedicular screw 50 is possible, releasing the two portions from each other, extracting them in a way substantially parallel to the axis of transpedicular screw 50 at lateral guide portion 10b, 10c.

This solution is particularly useful even if are used tubular screws are used guided through a Kirschner wire. In this case, in fact, it is possible to use drilling mask 100 for drilling a hole, then to put a Kirschner wire in the hole and maintaining in the pedicle the Kirschner wire during the extraction of the drilling mask. This way, the Kirschner wire can be used as guide for tubular screws or directly for guiding the screws (tubular or not).

In particular, reference element 21 is an element having a groove 21a arranged to engage and bridge spinous process 220. This way, reference element 21 embraces and engages spinous process 220. This allows positioning the inlet portion of drilling mask 100 with a correct orientation. This way, integrally to the other support elements 22, 23 in addition to the stability of drilling mask 100 also a control of its correct location is obtained.

In particular, as shown in Figs. 3 and 4, guide body 10 comprises two lateral guide portions 10b and 10c arranged at opposite sides with respect to reference portion 10a and equipped with a respective guide channel 25. Each lateral guide portion 10b and 10c is releasably connected to reference portion 10a, in order to move from the use configuration A (Fig.3) to the extraction configuration B (Fig.4). Guide body 10 in the use configuration A is substantially axisymmetrical with respect to spinous process 220. This way, drilling mask 100 besides assisting its own removal is modular and provides replacing the side portions 10b, 10c with other equivalent portions, for example responsive to the kind of transpedicular screw that can be implanted.

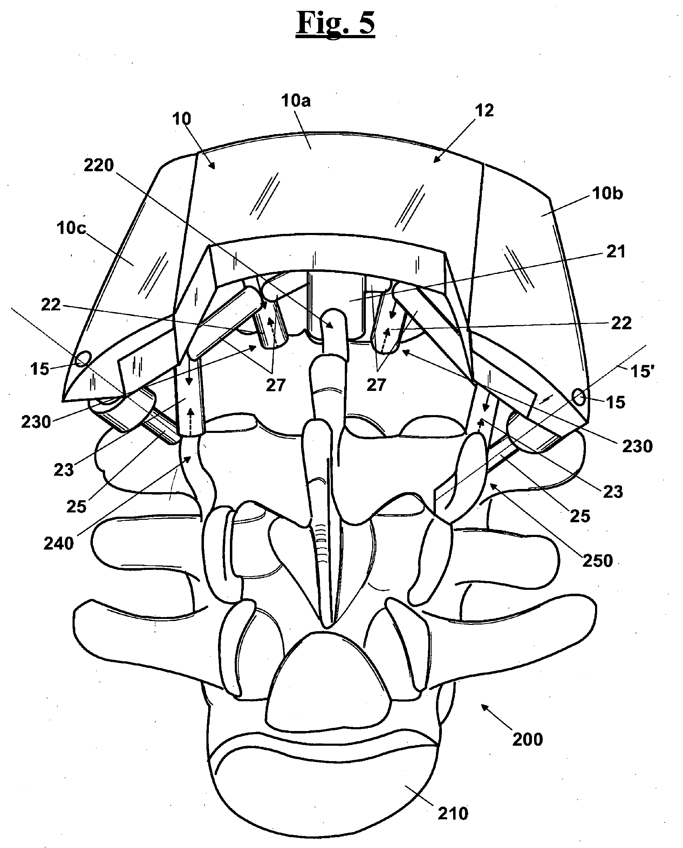

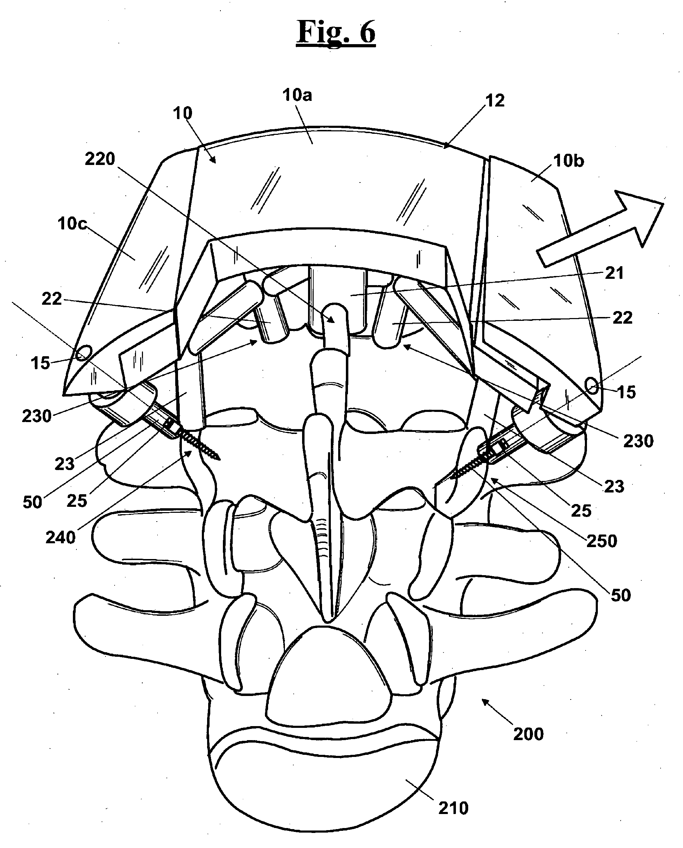

In a preferred exemplary embodiment, with reference to Figs. 5 and 6, support elements are provided adapted to contact side parts of spine section 200 comprising at least one couple of vertebral support elements 22 on the vertebral lamina 230 arranged substantially symmetrically with respect to spinous process 220, at least one couple of articular support elements 23 on the articular process 240, which are arranged substantially symmetrically with respect to spinous process 220 associated with guide channels 25 configured to be put at the pedicle 250. This way, drilling mask 100 presents a plurality of support elements in order to minimize the tissues to remove/detach and at the same time offer a steady and deterministic support, without false supports, i.e. such that all the support elements rest on a steady position of the spine section and not on the soft tissues or are raised therefrom.

In particular, each support element 22, 23 is orthogonal to the surface of the portion of bone on which it rests. This way, the compression force generated by the surgeon and a respective force of reaction are aligned with the axis of the relative support. (Fig. 5).

In addition, the same principle is valid also for guide channel 25 that is substantially orthogonal to the surface of the pedicle on which it rests.

To obtain the above described condition the resultant force of reaction on the support elements, including also the guide channels, and the force applied by the hand of the surgeon has to be zero, to avoid translations.

More in particular, the resultant force of reaction on the support elements has to fall within the vectorial space defined by the axes of the four support elements. The direction of the force applied by the surgeon must lay along the same axis of the resultant force of reaction on the support elements. Therefore also the direction of the force applied by the surgeon has to be contained in the vectorial space determined by the axes of the four stems.

This way, it is ensured that no translation of the mask.

In addition to the translation the condition of not rotating the mask has to be verified. This depends also from the point where the hand of the surgeon applies the force, as well as from its direction.

For achieving this condition, the moment of the forces calculated with respect to any pole must result zero. For example, pushing the mask only at a point, to obtain stability requires that the force is applied along a line that passes within the contact areas, as it occurs for a tripod located on a plane, where the centre of the mass must lay between the tripod feet.

In a simplified form of the drilling mask a lower number of lateral supports can be provided, for example a single articular support 23 at a lateral portion 10b, 10c can be provided associated with a vertebral support 22 located at opposite portion 10c, 10b, in order to give always a overall steady support to drilling mask 100.

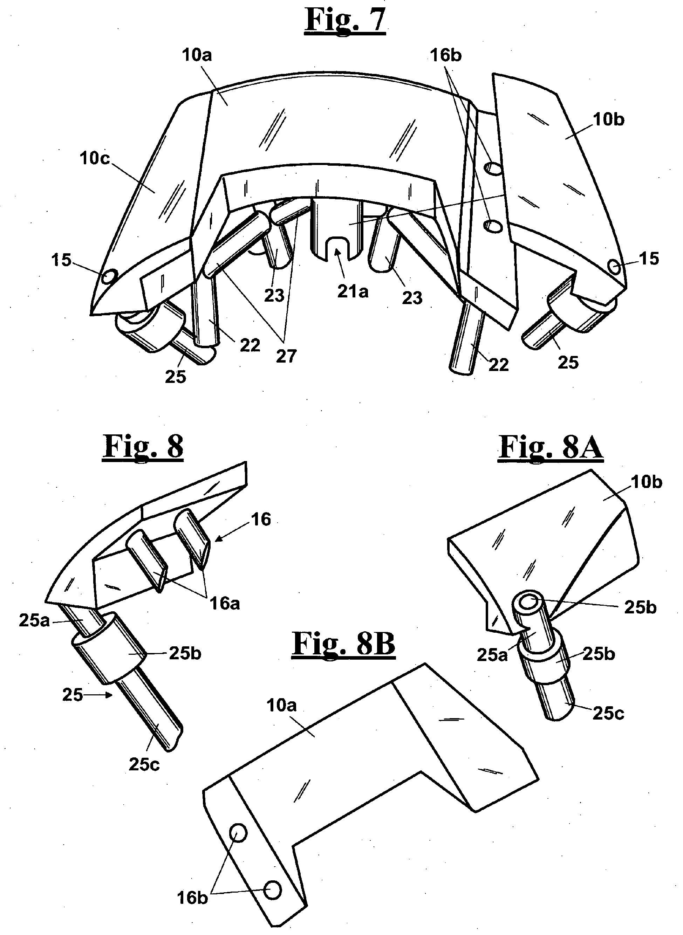

In addition, as shown in Fig. 7, reference element 21 and the couple of support elements 22 on the vertebral lamina 230, and the couple of support elements 23 on the articular process 240 in addition to guide channels 25, are connected to each other by connection elements 27, in order to form a support bar arranged to keep a precise relative position between the support elements, reference element 21 and guide channel 25. This way, the two couples of support elements 22, 23 provide a stability to the structure and allow the correct arrangement of the same since they provide a redundant reference for the support elements for determining wrong positioning due to the presence of residues of soft tissue. In other words, if for example drilling mask 100 is stable, but the surgeon finds that a support has moved upwards, it means that the positioning is not correct and then it is necessary to check any presence of a soft tissue under the other support elements. In fact, in the known solutions having only two support elements, in substance only three support points are present. This causes positioning errors if, even only under a single support, soft tissue parts are present. In these cases the surgeon can incorrectly think of having located correctly the mask, since all the support points are in contact with the tissue located underneath and there are not any oscillations thereof.

Even more in particular, as shown in Figs. 8 and 8B the mutual engagement means 16 comprises, in a possible exemplary embodiment, two pins 16a (Fig.8) arranged to fit in holes 16b made on reference portion 10a (Fig.8B), in order to connect to a relative lateral guide portion 10b, 10c. The pins 16a have an axis substantially parallel to the guide channel 25, in order to assist the extraction of the side portions and avoid undercut portions.

Alternatively, the above described pins can be made as removable pins 16a put in special through holes 16b' made on the lateral portion which allow locking with the respective hole 16b obtained in reference portion 21. The pins 16a may have a protruding head for assisting the extraction.

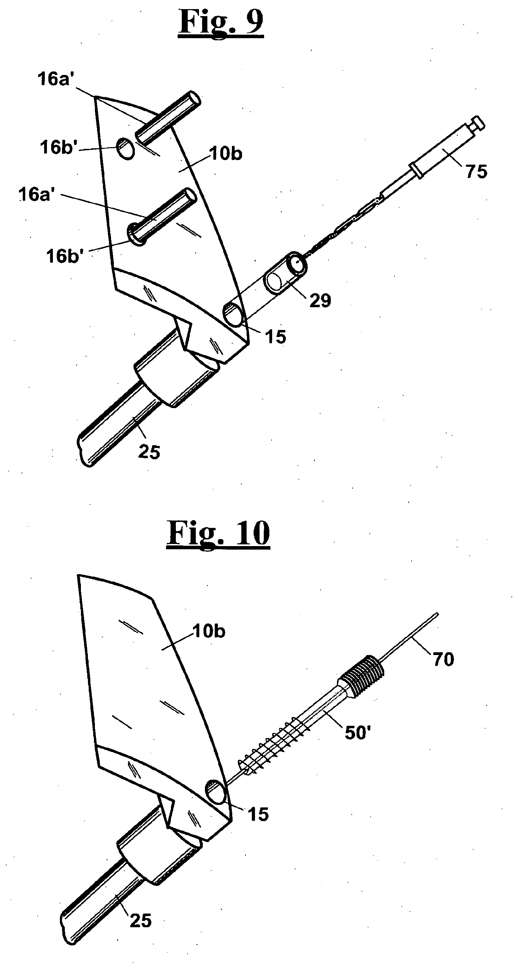

Figs. 8 and 8A show two different exemplary embodiments of the guide channel 25 obtained at lateral guide portions 10b, 10c. In particular, in the first exemplary embodiment of Fig. 8, guide portion 10b, 10c is adapted to guide a drill bit 75, as also shown in Fig. 9, and provides a first zone of engaging drill bit 25a, a central stiffening zone 25b and a end zone 26c that rests at the pedicle. The diameter of guide channel 25 is in this case proportioned to the diameter of the drill bit. In the exemplary embodiment of Fig. 8A a guide channel 25 is provided with increase diameter for guiding transpedicular screw 50.

Alternatively, as shown in Figs. 9 and 10, a guide channel 25 can be provided with a reduction element 29, in particular a bush, configured to be put in guide channel 25, in order to reduce the diameter for the step of drilling the pedicle (Fig.9).

After drilling it is possible to remove the reduction bush 29 for introduction of the transpedicular screw. This way, it is possible to adapt the guide channel responsive to the diameter of the transpedicular screw used, in order to obtain a higher introduction precision.

From a structural viewpoint, drilling mask 100 is obtained starting by data obtained by an imaging means that measure a volumetric image of spine section 200 and a CAD modelling means, that model drilling mask 100 on the basis of the volumetric image, such that reference element 21 on spinous process 220, the couple of vertebral support elements 22 on the vertebral lamina 230, and the couple of articular support elements 23 on the articular process 240 fit to the bone profile 260, in order to obtain a support stability and a precision of positioning for the implant of transpedicular screw 50. In particular, the imaging means is adapted to obtain radiographic volumetric images, in particular computed tomography or magnetic resonance images.

This way, drilling mask 100 can be located on the corresponding vertebra of the patient after having made a surgical access of size similar to that made for carrying out a traditional operation under fluoroscopy or freehand operation. The couple of vertebral support elements 22 on the vertebral lamina 230 and on the articular process 240 of the mask are located after having displaced the muscle tissue located underneath, whereas for the reference spinous element 21 no additional steps are necessary, with respect to the traditional operation. Once effected the correct arrangement of drilling mask 100 and then of the guide channel 25, the surgeon can proceed, according to the screws used, for drilling the pedicle 250 using a Kirschner wire and then causing a tubular screw to slide on it, or to proceed directly to guide of screw, by arranging the channel for implanting screws.

In particular, the CAD modelling means provides a means for segmenting the model of drilling mask 100 into a plurality of cross sections so that reference element 21 and the support elements have a profile that is the negative of the bone profile 260 obtained by the segmentation, thus contributing to increase the stability.

The foregoing description of specific exemplary embodiments will so fully reveal the invention according to the conceptual point of view, so that others, by applying current knowledge, will be able to modify and/or adapt in various applications the specific exemplary embodiments without further research and without parting from the invention, and, then it is meant that such adaptations and modifications will have to be considered as equivalent to the specific embodiments. The means and the materials to realise the different functions described herein could have a different nature without, for this reason, departing from the field of the invention. It is to be understood that the phraseology or terminology that is employed herein is for the purpose of description and not of limitation.

## Claims

1. A drilling mask (100) for implanting a transpedicular screw (50) to a spine section (200), said spine section (200) having a vertebral body (210) with a spinous process (220), a vertebral lamina (230) and a articular process (240) with a relative pedicle (250), said drilling mask (100) comprising:
- a guide body (10) having a distal surface (12) and a proximal surface (14), opposite to said distal surface (12) and suitable for facing towards said spine section (200),
- a reference element (21) obtained on said proximal surface (14) and arranged to contact said spinous process (220),
- a guide channel (25) having an inlet (15) in said distal surface (12) and arranged to work as drilling guide for drilling said pedicle (250) and implanting said transpedicular screw (50);
- a plurality of support elements (22, 23) made on said proximal surface (14), associated with said reference element (21) and arranged to contact side parts of said spine section (200),
wherein said guide body (10) comprises:
- at least one reference portion (10a), comprising said reference element (21), configured to be put at said spinous process (220), and
- a lateral guide portion (10b, 10c), cooperating with said reference portion (10a), and comprising said guide channel (25) for drilling said pedicle (250), where said reference portion (10a) and said lateral guide portion (10b, 10c) are mutually coupled to each other through releasable mutual engagement means (16), in such a way that said guide body can, when mounted to said spire section, move from a use configuration (A), where said reference portion (10a) and said lateral guide portion (10b, 10c) are connected to each other for introducing said transpedicular screw (50), to an extraction configuration (B), where said reference portion (10a) and said lateral guide portion (10b, 10c) are disassembled from each other
**characterised in that** said reference portion (10a) and said lateral guide portion (10b, 10c) are disassembled from each other for removal of said lateral guide portion (10b, 10c) from said reference portion in a direction substantially parallel with respect to an axis (15') of said guide channel (25) and for removal of said reference portion (10a) from said spine section in a direction substantially parallel to said spinous process (220),
**and in that**
said reference element (21) is an element having a groove (21a) arranged to engage bridging said spinous process (220).

2. A drilling mask (100), according to claim 1, wherein said guide body (10) comprises two lateral guide portions (10b, 10c) arranged at opposite sides with respect to said reference portion (10a) and equipped with a respective guide channel (25), each of said lateral guide portions (10b, 10c) being releasably connected to said reference portion (10a), in such a way that it can pass from said use configuration (A) to said extraction configuration (B).

3. A drilling mask (100), according to claim 1, wherein said plurality of support elements (22, 23) is arranged to contact side parts of said spine section (200) and comprise at least one couple of vertebral support elements (22) on said vertebral lamina (230) arranged substantially symmetrically with respect to said spinous process (220), at least one couple of articular support elements (23) on said articular processes (240), which are arranged substantially symmetrically with respect to said spinous process (220), associated with said guide channels (25) configured to be put at a respective pedicle (250).

4. A drilling mask (100), according to claim 1, wherein each support element (22, 23) is substantially orthogonal to the surface of the bone profile on which it rests in use.

5. A drilling mask (100), according to claim 1, wherein said guide channel (25) is substantially orthogonal to the surface of the pedicle on which it rests.

6. A drilling mask (100), according to claim 1 and 3, where said reference element (21) and said couple of vertebral support elements (22) on said vertebral lamina (230) and articular support elements (23) on said articular processes (240) and said guide channel (25) are connected to each other by connection elements (27), in order to form a support bar arranged to keep a precise relative position between said support elements (22, 23), said reference element (21) and said guide channel (25).

7. A drilling mask (100), according to claim 3, wherein said guide channel (25) comprises a reduction element (29), in particular a bush, configured to be put in said guide channel (25), in order to reduce the diameter for use of transpedicular screws that enter precisely in said reduction element (29).

8. A drilling mask (100), according to claim 3, **characterized in that** the following features are also provided:
- an imaging means for obtaining said reference element (21) on said spinous process (220), said vertebral support elements (22) on said vertebral lamina (230), and said articular support elements (23) on said articular processes (240) of said drilling mask, said imaging means arranged to measure a volumetric image of said spine section (200);
- a CAD modelling means for modelling said drilling mask (100) on the basis of said volumetric image, in such a way that said reference element (21) on said spinous process (220), said vertebral support elements (22) on said vertebral lamina (230), and said articular support elements (23) on said articular processes (240) fit the bone profile, in such a way to obtain a support stability for implanting said transpedicular screw (50), in particular said imaging means is arranged to obtain radiographic volumetric images, in particular computed tomography or magnetic resonance.

9. A drilling mask (100), according to claim 8, wherein said CAD modelling means provides a means for segmenting said model of drilling mask (100) into a plurality of cross sections in such a way that said reference element (21) and said support elements (22, 23) have a profile that is the negative of the bone profile obtained by said segmentation, thus contributing to increase the stability.

## Patentansprüche

1. Bohrmaske (100) zur Implantation einer transpedikulären Schraube (50) in einen Wirbelsäulenabschnitt (200), wobei der Wirbelsäulenabschnitt (200) über einen Wirbelkörper (210) mit einem Dornfortsatz (220), einer Bogenplatte (230) und einem Gelenkfortsatz (240) mit einer entsprechenden Wurzel (250) verfügt, die Bohrmaske (100) umfassend:
- einen Führungskörper (10) mit einer distalen Oberfläche (12) und einer proximalen Oberfläche(14), die der distalen Oberfläche (12) gegenüber liegt und dafür geeignet ist, hin zum Wirbelsäulenabschnitt (200) zu zeigen,
- ein Referenzelement (21), das auf der proximalen Oberfläche (14) erlangt wird und so angeordnet wird, dass es den Dornfortsatz (220) berührt,
- einen Führungskanal (25) mit einem Eingang (15) in der distalen Oberfläche (12) und so angeordnet, dass er als Bohrführung zum Bohren der Wurzel (250) und zur Implantation der transpedikulären Schraube (50) wirkt;
- eine Vielzahl von Stützelementen (22, 23), die an der proximalen Oberfläche (14) erstellt werden, die mit dem Referenzelement (21) assoziiert sind und so angeordnet sind, dass sie die seitlichen Teile des Wirbelsäulenabschnitts (200) berühren,
wobei der Führungskörper (10) Folgendes umfasst:
- mindestens einen Referenzteil (10a), umfassend das Referenzelement (21), konfiguriert, um auf den Dornfortsatz (220) aufgelegt zu werden, und
- einen seitlichen Führungsteil (10b, 10c), der mit dem Referenzteil (10a) zusammenarbeitet und den Führungskanal (25) zum Bohren der Wurzel (250) umfasst, wobei der Referenzteil (10a) und der seitliche Führungsteil (10b, 10c) so gegenseitig über freigebbare gemeinsame Verbindungsmittel (16) miteinander verbunden sind, dass sich der Führungskörper, wenn am Wirbelsäulenabschnitt befestigt, von einer Verwendungskonfiguration (A), bei der der Referenzteil (10a) und der seitliche Führungsteil (10b, 10c) miteinander verbunden sind, um die transpedikuläre Schraube (50) einzuführen, in eine Extraktionskonfiguration (B), bei der der Referenzteil (10a) und der seitliche Führungsteil (10b, 10c) voneinander getrennt sind, bewegen kann,
**dadurch gekennzeichnet, dass** der Referenzteil (10a) und der seitliche Führungsteil (10b, 10c) zur Entfernung des seitlichen Führungsteils (10b, 10c) vom Referenzteil in einer Richtung, die im Wesentlichen parallel zu einer Achse (15') des Führungskanals (25) liegt, und zur Entfernung des Referenzteils (10a) vom Wirbelsäulenabschnitt in einer Richtung, die im Wesentlichen parallel zum Dornfortsatz (220) liegt, voneinander getrennt werden,
**und dadurch, dass**
das Referenzelement (21) ein Element mit einer Rille (21a) ist, die so angeordnet ist, dass sie zur Überbrückung des Dornfortsatzes (220) ineinander greift.

2. Bohrmaske (100) nach Anspruch 1, wobei der Führungskörper (10) zwei seitliche Führungsteile (10b, 10c) umfasst, die an gegenüberliegenden Seiten in Bezug auf den Referenzteil (10a) angeordnet sind und mit einem entsprechenden Führungskanal (25) ausgestattet sind, wobei jeder der seitlichen Führungsteile (10b, 10c) so freigebbar mit dem Referenzteil (10a) verbunden ist, dass er von der Verwendungskonfiguration (A) in die Extraktionskonfiguration (B) wechseln kann.

3. Bohrmaske (100) nach Anspruch 1, wobei die Vielzahl der Stützelemente (22, 23) so angeordnet ist, dass sie die seitlichen Teile des Wirbelsäulenabschnitts (200) berührt, und mindestens ein Paar von Wirbelstützelementen (22) an der Bogenplatte (230), die im Wesentlichen symmetrisch in Bezug auf den Dornfortsatz (220) angeordnet ist, und mindestens ein Paar Gelenkstützelemente (23) an den Gelenkfortsätzen (240), die im Wesentlichen symmetrische in Bezug auf den Dornfortsatz (220) angeordnet sind, assoziiert mit den Führungskanälen (25), die zum Auflegen auf eine entsprechende Wurzel (250) konfiguriert sind, umfasst.

4. Bohrmaske (100) nach Anspruch 1, wobei jedes Stützelement (22, 23) im Wesentlichen orthogonal zur Oberfläche des Knochenprofils, auf der es bei der Verwendung liegt, ist.

5. Bohrmaske (100) nach Anspruch 1, wobei der Führungskanal (25) im Wesentlichen orthogonal zur Oberfläche der Wurzel, auf der er liegt, ist.

6. Bohrmaske (100) nach Anspruch 1 und 3, wobei das Referenzelement (21) und das Paar von Wirbelstützelement (22) an der Bogenplatte (230) und die Gelenkstützelemente (23) an den Gelenkfortsätzen (240) und dem Führungskanal (25) über Verbindungselemente (27) miteinander verbunden sind, um einen Stützbalken zu bilden, der so angeordnet ist, dass eine genaue relative Position zwischen den Stützelementen (22, 23), dem Referenzelement (21) und dem Führungskanal (25) beibehalten wird.

7. Bohrmaske (100) nach Anspruch 3, wobei der Führungskanal (25) ein Reduzierelement (29), insbesondere eine Hülse, umfasst, das zum Einsetzen in den Führungskanal (25) konfiguriert ist, um den Durchmesser zur Verwendung von transpedikulären Schrauben, die genau in das Reduzierelement (29) eindringen, zu reduzieren.

8. Bohrmaske (100) nach Anspruch 3, **dadurch gekennzeichnet, dass** die folgenden Funktionen bereitgestellt werden:
- eine Bildgebungsvorrichtung zur Erlangung des Referenzelements (21) am Dornfortsatz (220), des Wirbelstützelements (22) an der Bogenplatte (230) und der Gelenkstützelemente (23) an den Gelenkfortsätzen (240) der Bohrmaske, wobei die Bildgebungsvorrichtung so angeordnet ist, dass sie ein volumetrisches Bild der Wirbelsäulenabschnitts (200) misst;
- eine CAD-Modellierungsvorrichtung zur Modellierung der Bohrmaske (100) auf Grundlage des volumetlischen Bilds, so dass das Referenzelement (21) am Dornfortsatz (220), die Wirbelstützelemente (22) an der Bogenplatte (230) und die Gelenkstützelemente (23) an den Gelenkfortsätzen (240) dem Knochenprofil entsprechen, so dass eine Stützstabilität zur Implantation der transpedikulären Schraube (50) erlangt wird, insbesondere ist die Bildgebungsvorrichtung so angeordnet, dass radiographische volumetrische Bilder erlangt werden, insbesondere Computertomographie oder Magnetresonanz.

9. Bohrmaske (100) nach Anspruch 8, wobei die CAD-Modellierungsvorrichtung eine Vorrichtung zur Zerlegung des Modells der Bohrmaske (100) in eine Vielzahl von Querschnitten bereitstellt, so dass das Referenzelement (21) und die Stützelemente (22, 23) ein Profil haben, das dem Negativ des Knochenprofils entspricht, das durch die Zerlegung erhalten wurde, wodurch zur Erhöhung der Stabilität beigetragen wird.

## Revendications

1. Masque de perçage (100) permettant d'implanter une vis transpédiculaire (50) sur une section de colonne vertébrale (200), ladite section de colonne vertébrale (200) présentant un corps vertébral (210) muni d'une apophyse épineuse (220), une lame vertébrale (230) et une apophyse articulaire (240) munie d'un pédicule respectif (250), ledit masque de perçage (100) comprenant:
- un corps de guidage (10) ayant une surface distale (12) et une surface proximale (14), opposée à ladite surface distale (12) et adaptée pour faire face à ladite section de colonne vertébrale (200),
- un élément de référence (21) prévu sur ladite surface proximale (14) et disposé pour toucher ladite apophyse épineuse (220),
- un canal de guidage (25) ayant un orifice d'admission (15) dans ladite surface distale (12) et disposé pour faire office de guide de perçage pour percer ledit pédicule (250) et implanter ladite vis transpédiculaire (50);
- une pluralité d'éléments supports (22,23) prévus sur ladite surface proximale (14), associés audit élément de référence dit (21) et organisés pour toucher les parties latérales de ladite section de colonne vertébrale (200),
dans lequel ledit corps de guidage (10) comprend:
- au moins une portion de référence (10a), comprenant ledit élément de référence (21), configuré pour être placé au niveau de ladite apophyse épineuse (220), et
- une portion de guidage latérale (10b, 10c), coopérant avec ladite partie de référence (10a), et comprenant ledit canal de guidage (25) pour percer ledit pédicule (250), où ladite portion de référence (10a) et ladite portion de guidage latérale (10b, 10c) sont mutuellement accouplées l'une à l'autre grâce à des moyens d'engagement mutuels et libérables (16), de telle manière que ledit corps de guidage, une fois monté sur ladite section de colonne vertébrale, puisse passer d'une configuration d'utilisation (A) dans laquelle ladite portion de référence (10a) et ladite portion de guidage latérale (10b, 10c) sont connectées l'une à l'autre pour introduire ladite vis transpédiculaire (50), à une configuration d'extraction (B) dans laquelle ladite portion de référence (10a) et ladite portion de guidage latérale (10b, 10c) sont séparées l'une de l'autre,
**caractérisé en ce que** ladite portion de référence (10a) et ladite portion de guidage latérale (10b, 10c) sont séparées l'une de l'autre pour permettre la dépose de ladite portion de guidage latérale (10b, 10c) par rapport à de ladite partie de référence dans une direction sensiblement parallèle à un axe (15') dudit canal de guidage (25) et la dépose de la portion de référence (10a) de ladite section de colonne vertébrale dans une direction sensiblement parallèle à ladite apophyse épineuse (220),
**et en ce que** ledit élément de référence (21) est un élément ayant une gorge (21a) prévue pour s'engager par pontage dans ladite apophyse épineuse (220).

2. Masque de perçage (100), selon la revendication 1, dans lequel ledit corps de guidage (10) comprend deux portions de guidage latérales (10b, 10t) disposées sur des côtés opposés par rapport à ladite portion de référence (10a) et équipées d'un canal de guidage respectif (25), chacune des portions de guide latérales (10b, 10c) étant connectée de manière libérable à ladite portion de référence (10a), de manière à pouvoir passer de ladite configuration d'utilisation (A) à ladite configuration d'extraction (B).

3. Masque de perçage (100), selon la revendication 1, dans lequel ladite pluralité d'éléments supports (22, 23) est aménagée pour toucher des parties latérales de ladite section de colonne vertébrale (200) et comprendre au moins un ou deux d'éléments supports vertébraux (22) sur ladite lame vertébrales (230) disposées sensiblement de manière symétrique par rapport à ladite apophyse épineuse (220), au moins une paire d'éléments supports articulaires (23) sur ladite apophyse articulaire (240), qui sont disposées sensiblement de manière symétrique par rapport à ladite apophyse épineuse (220), associés avec lesdits canaux de guidage (25) configurés pour être placés au niveau d'un pédicule respectif (250).

4. Masque de perçage (100), selon la revendication 1, dans lequel chaque élément support (22, 23) est sensiblement orthogonal par rapport à la surface du profil osseux sur laquelle il repose en cours d'utilisation.

5. Masque de perçage (100), selon la revendication 1, dans lequel ledit canal de guidage (25) est sensiblement orthogonal par rapport à la surface du pédicule sur laquelle il repose.

6. Masque de perçage (100), selon la revendication 1 et la revendication 3, dans lequel l'élément de référence (21) et ledit couple d'éléments supports vertébraux (22) sur ladite lame vertébrale (230) et d'éléments supports articulaires (23) sur ladite apophyse articulaire (240) et ledit canal de guidage (25) sont reliés les uns aux autres par des éléments de connexion (27), pour former une barre de support disposée pour maintenir une position relative précise entre lesdits éléments supports (22, 23), ledit élément de référence (21) et ledit canal de guidage (25).

7. Masque de perçage (100), selon la revendication 3, dans lequel ledit canal de guidage (25) comprend un élément de réduction (29), en particulier une douille, configurée pour être placé dans ledit canal de guidage (25), afin de réduire le diamètre pour utiliser la vis transpédiculaire pénétrant avec précision ledit élément de réduction (29).

8. Masque de perçage (100), selon la revendication 3, **caractérisé en ce que** les fonctions suivantes sont également prévues :
- un moyen d'imagerie pour l'obtention dudit élément de référence (21) sur ladite apophyse épineuse (220), ledit élément support vertébral (22) sur ladite lame vertébrale (230) et des éléments supports articulaires (23) sur lesdites apophyses articulaires (240) dudit masque de perçage, ledit moyen d'imagerie disposée pour mesurer une image volumétrique de ladite section de colonne vertébrale (200);
- un moyen de modélisation CAO pour modéliser ledit masque de perçage (100) sur la base de ladite image volumétrique, de telle sorte que ledit élément de référence (21) sur ladite apophyse épineuse (220), lesdits éléments supports vertébraux (22) sur ladite lame vertébrale (230), et lesdits éléments supports articulaires (23) sur lesdites apophyses articulaires (240) correspondent au profil osseux, de façon à obtenir un support stable pour implanter ladite vis transpédiculaire (50), en particulier, ledit moyen d'imagerie est configuré pour obtenir des images volumétriques radiographiques, en particulier par tomographie informatisée ou par résonance magnétique.

9. Masque de perçage (100), selon la revendication 8, dans lequel ledit moyen de modélisation CAO constitue un moyen pour segmenter ledit modèle de masque de perçage (100) en une pluralité de sections transversales de telle sorte que ledit élément de référence (21) et lesdits éléments supports (22, 23) aient un profil correspondant au négatif du profil osseux obtenu par ladite segmentation, contribuant ainsi à accroître la stabilité.
